Europäisches Patentamt
European Patent Office
Office européen des brevets

(11) Publication number: 0 314 008 A2

(12) EUROPEAN PATENT APPLICATION

(21) Application number: 88117557.4

(22) Date of filing: 21.10.88

(51) Int. Cl.4: C07D 295/02 , C07D 295/18 , A61K 31/54 , A61K 31/55 , A61K 31/535

Claims for the following Contracting States: ES + GR.

(30) Priority: 28.10.87 IT 2242387

(43) Date of publication of application: 03.05.89 Bulletin 89/18

(84) Designated Contracting States: AT BE CH DE ES FR GB GR IT LI LU NL SE

(71) Applicant: MEDIOLANUM FARMACEUTICI s.r.l.
Via S.G. Cottolengo, 31
I-20143 Milano(IT)

(72) Inventor: Meroni, Carlo
Via Giovanni XXIII, 11
Meda (Milan)(IT)
Inventor: Maiorana, Stefano
Via Don C. Porro, 6
Milan(IT)
Inventor: Rugarli, Pier Luigi
Via Federico Chopin, 89
Milan(IT)
Inventor: Pagella, Pier Giuseppe
Frazione Capraglia, 56
Isola S. Antonio Alessandria(IT)

(74) Representative: Gervasi, Gemma
NOTARBARTOLO & GERVASI Srl Viale
Bianca Maria 33
I-20122 Milan(IT)

(54) N-substituted aliphatic heterocyclic compounds able to facilitate the passage of active substances through physiological barriers.

(57) N-substituted aliphatic heterocyclic compounds having the following general formula:

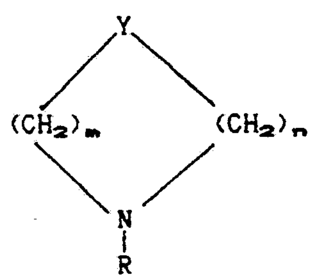

(I)

where Y is $CH_2$, S, S>0 or 0;
m and n are whole numbers between 0 and 10 and such that their sum is a whole number between 3 and 10;
R is a $C_6$-$C_{20}$ linear or branched alkyl or an acyl of general formula

EP 0 314 008 A2

- $\overset{O}{\overset{\parallel}{C}}$ -$R_1$ in which $R_1$ is a $C_5$-$C_{19}$ linear or branched alkyl; and a process for their production.

Said compounds have low toxicity, and high capacity in facilitating the passage of active substances through physiological barriers.

Said compounds also possess anti-inflammatory and antiallergic activity.

FIG 1

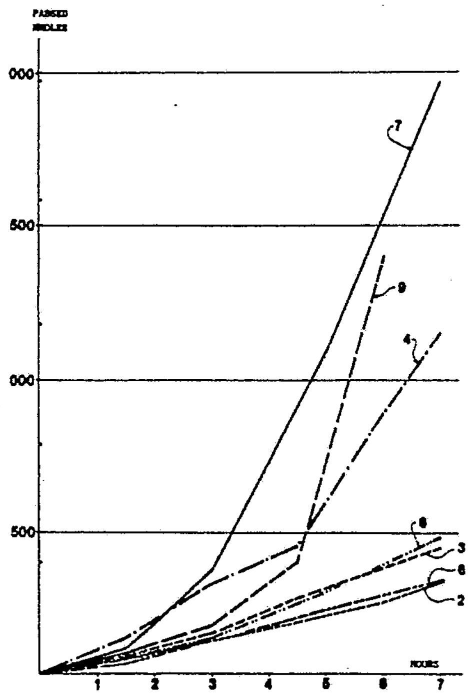

# N-SUBSTITUTED ALIPHATIC HETEROCYCLIC COMPOUNDS ABLE TO FACILITATE THE PASSAGE OF ACTIVE SUBSTANCES THROUGH PHYSIOLOGICAL BARRIERS

## Field of the invention

This invention relates to a series of non-toxic N-substituted aliphatic heterocyclic compounds useful in facilitating the passage of active substances through physiological barriers. Said compounds also possess anti-inflammatory and antiallergic activity.

## Prior art

Numerous substances have been used as vehicles in the passage of active substances, particularly through the skin. These include dimethylsulphoxide, various amines including N-substituted morpholines and diisopropanolamines (N. Baker and J. Hadgraft, Int. J. Pharmac. 8, 1981, 193-202), amides of cyclic amines (D. Mirejovsky and H. Takruri, J. Pharm. Sci. 75, 1986, 1089-1093) and finally the substituted azacycloalkan-2-ones-1- which are described in US patents 3,989,816, 4,316,893 and 4,405,616 .

## Summary of the invention

We have now discovered a new series of N-substituted aliphatic heterocyclic compounds which are much more effective than known substances in facilitating the passage of active substances through the various physiological barriers both of the animal kingdom (thus facilitating for example the passage of various medicaments) and of the vegetable kingdom (thus facilitating for example the dyeing of natural fibres).

The compounds of the present invention have the following general formula:

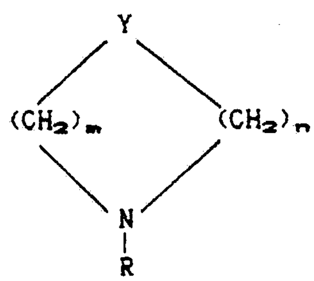

(I)

where Y is $CH_2$, S, S->O or O;
m and n are whole numbers between 0 and 10 and such that their sum is a whole number between 3 and 10;
R is a $C_6$-$C_{20}$ linear or branched alkyl or an acyl of general formula
$-\overset{O}{\overset{||}{C}}-R_1$, in which $R_1$, is a $C_5$-$C_{19}$ linear or branched alkyl.

The invention also relates to a process for preparing the compounds (I) consisting of treating the compound

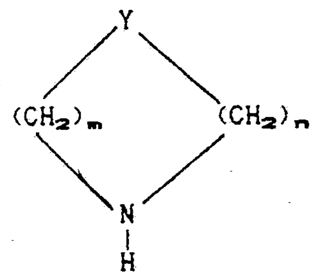

(II)

in an organic solvent means with NaH and then with an RX alkyl halide, or treating (II) with an $R_1-\overset{O}{\overset{\|}{C}}-X$ acyl halide in the presence of an alkaline substance, where R and $R_1$ have the aforesaid meanings and X is a halogen.

The invention also relates to pharmaceutical compositions containing at least one compound (I) for the purpose of facilitating passage of active substances through physiological barriers.

The invention further relates to pharmaceutical compositions containing at least one compound (I) for external topical use for anti-inflammatory and antiallergic purposes.

## Detailed description of the invention

The compounds of general formula (I) where R = alkyl as heretofore defined are prepared by treating the compounds of general formula (II) with NaH in an organic solvent under boiling conditions. The alkyl halide RX dissolved in an organic solvent is then gradually added to the mixture which is then reheated to boiling. The organic solvents which can be used include benzene, xylene, toluene, dioxane, diglyme and tetrahydrofuran. Toluene is preferably used.

The alkyl halide can be a bromide, chloride or iodide. Alkyl bromide is preferred.

The obtained compound can be oxidised to obtain the compound (I) where Y = S->O. This oxidation is effected by adding a solution in $CH_3OH$ of said compound obtained as described heretofore to an aqueous $NaIO_4$ solution maintained at a temperature of between -5 and +10° C.

To prepare compounds of general formula (I) where R = acyl as heretofore defined, the compounds of general formula (II) are dissolved in an organic solvent and sodium bicarbonate or another inorganic base such as sodium carbonate is added, after which the acyl halide $R_1-\overset{O}{\overset{\|}{C}}-X$ dissolved in an organic solvent is added to this mixture while maintaining the temperature between 10 and 20° C. The reaction is then carried out at room temperature.

The solvents which can be used include benzene, toluene, xylene, chloroform, methylene chloride, ethyl ether, diisopropyl ether and petroleum ether. Prefereably benzene and methylene chloride are used. The acyl halide is preferably an acyl chloride.

The compounds of the present invention have the ability to facilitate passage through membranes to a considerably better extent than known substances used for the same purpose.

In addition, the compounds of the present invention possess anti-inflammatory and antiallergic activity. They also have a low degree of toxicity.

The following examples of the preparation and application of the compounds according to the invention are given by way of non-limiting illustration only.

## EXAMPLE 1: 4-dodecylthiomorpholine

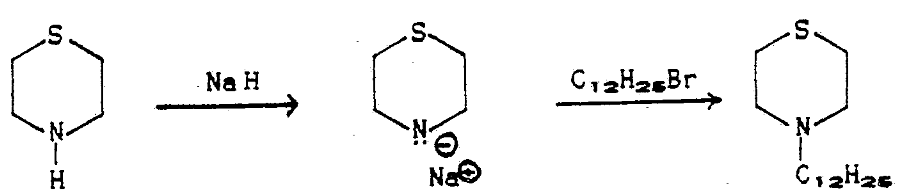

1.2 g (50 mmoles) of NaH (98%) are suspended in 100 ml of anhydrous toluene in a 250 ml 3-neck flask fitted with a magnetic stirrer, thermometer and reflux condenser.

4.6 g (44.5 mmoles) of thiomorpholine dissolved in 15 ml of toluene are dripped into said suspension over 30 minutes and the mixture heated under reflux for about 1 hour. 11.1 g (44.5 mmoles) of n-dodecylbromide dissolved in toluene are then dripped in over 20 minutes, after which the mixture is left to react under boiling conditions for about 25 hours.

The progress of the reaction is monitored by TLC ($SiO_2$, $CH_2Cl_2$:$CH_3OH$ ratio 10:1) displaying the spots by $I_2$.

After 25 hours the conversion is practically complete. The unreacted sodium hydride and salts are then filtered off and the toluene evaporated under reduced pressure. The residue is taken up in ethyl ether, the solution washed several times with water to remove the unreacted thiomorpholine residue, dried with $Na_2SO_4$ and the ethyl ether evaporated. 10 g of product are obtained (M.W. 271.53, yield 82%) in the form of a yellowish solid melting at ambient temperature.

Should the water washes be insufficient to remove the unreacted thiomorpholine, the crude reaction product is chromatographed on $SiO_2$ using as effluent firstly $CH_2Cl_2$ and then adding $CH_3OH$ to obtain a final $CH_2Cl_2$:$CH_3OH$ ratio of 10:1.

N.M.R. ($CDCl_3$, TMS): $\delta$ 0.9 (t, 3H, $CH_{3alkyl}$);$\delta$1.3 (s, 20H, $-(CH_2)_{10}$-alkyl),$\delta$2.35 (t, 2H,

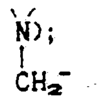

$\delta$2.7 (s, 8H, thiomorpholine ring)

| Elementary analysis: | CALCULATED | FOUND |
|---|---|---|
| C | 70.85 | 70.63 |
| H | 12.18 | 12.11 |
| N | 5.17 | 5.15 |

EXAMPLE 2: 4-dodecylthiomorpholine-1-oxide

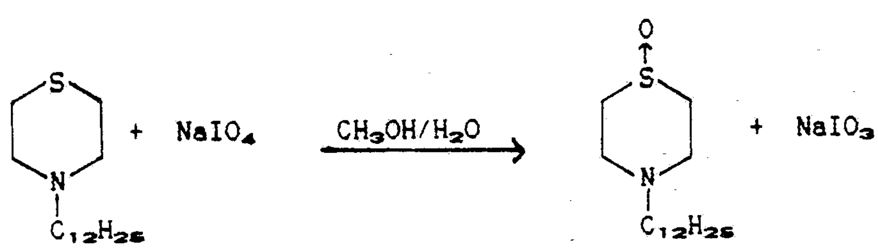


23.2 ml (11.6 mmoles) of a 0.5 molar aqueous solution of $NaIO_4$ are placed in a 100 ml 2-neck flask fitted with a magnetic stirrer and thermometer and kept at 0° C with ice.

3 g (11.05 mmoles) of 4-dodecylthiomorpholine dissolved in 20 ml of $CH_3OH$ are slowly added to this solution.

The progress of the reaction (maintained rigorously at 0° C and under energetic agitation) is monitored by TLC ($SiO_2$, $CH_2Cl_2$:$CH_3OH$ 10:1) displaying the spots by $I_2$.

After 25 hours the conversion is complete. The precipitate is filtered off and is carefully washed on the filter with $CH_2Cl_2$.

The organic solution is washed several times with water and then dried with $Na_2SO_4$. The $CH_2Cl_2$ is then evaporated under reduced pressure and the product separated in the form of micro-crystalline white

powder.

2.5 g of 4-dodecylthiomorpholine-1-oxide (M.W. 287.53) are obtained with a yield of 79%.

Should the relative sulphone form during the course of the reaction (detectable by TLC under the described conditions as a spot at lower Rf), the products are separated by chromatography on $SiO_2$ - (eluents $CHCl_3$:$CH_3OH$ ratio of 10:1).

M.P. 84.5-86.1 °C.

N.M.R. ($CDCl_3$, TMS): $\delta$ 0.85 (t, 3H, $CH_3$alkyl);$\delta$1.25 (s. 20H, -$(CH_2)_{10}$-alkyl),$\delta$2.4 (t, 2H,

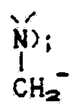

$\delta$2.55-3.2 (m, 8H, thiomorpholine ring)

I.R. (nujol); $\nu_{S\rightarrow O}$ 1020 $cm^{-1}$

| Elementary analysis: | CALCULATED | FOUND |
|---|---|---|
| C | 66.90 | 66.72 |
| H | 11.50 | 11.35 |
| N | 4.88 | 4.81 |

EXAMPLE 3: 4-lauroylthiomorpholide

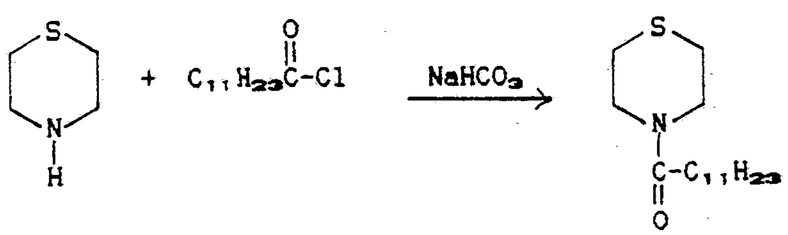


4.6 g (44.54 mmoles) of thiomorpholine are dissolved in 80 ml of benzene in a 250 ml flask fitted with a thermometer and magnetic stirrer and cooled to 10 °C in an ice-water bath, and 4.1 g (49 mmoles) of sodium bicarbonate are suspended in the solution. 9.75 g (10.3 ml, 44.55 mmoles) of lauroyl chloride are dissolved separately in 20 ml of benzene and this solution is dripped very slowly into the thiomorpholine solution.

During the addition a voluminous precipitate (thiomorpholine hydrochloride) forms but disappears as the reaction proceeds. The temperature is kept constant by means of the water bath (the reaction is exothermic).

The progress of the reaction is followed by TLC($SiO_2$, $CHCl_3$: $CH_3OH$ ratio of 10:0.2) displaying the spots by exposure to $I_2$ vapour.

On completion of the dropwise addition (1 hour and 30 minutes) the reaction mixture is kept stirred at ambient temperature for a further 30 minutes.

It is then poured into a separator funnel in which it is washed several times with water and acidified water to remove any unreacted thiomorpholine residues. The benzene solution is then dried with $Na_2SO_4$ and, after filtering off the sulphate, the benzene is evaporated under reduced pressure. The product is separated (12 g, M.W. 285.46, yield 94%) in the form of a dense oily liquid which solidifies at ambient temperature after a certain time.

Further purification can be carried out by chromatographing the product on $SiO_2$ (eluents $CHCl_3$:$CH_3OH$ 10:0.2).

M.P. 38-39° C.
N.M.R. ($CDCl_3$, TMS): $\delta$ 0.9 (t, 3HJ, $CH_3$alkyl);$\delta$1.35 (s, 18H, $-(CH_2)_9$-alkyl),$\delta$2.3 (t, 2H, $-\underset{\underset{O}{\|}}{C}-CH_2-$);$\delta$2.55-2.85 (m, 4H, thiomorpholine ring, H adjacent to S);$\delta$3,65-4.00 (m, 4H, thiomorpholine ring, H adjacent to N)
I.R. (liquid film): $\nu_{C=O}$ 1645 $cm^{-1}$

| Elementary analysis: | CALCULATED | FOUND |
|---|---|---|
| C | 67.37 | 67.22 |
| H | 10.88 | 10.65 |
| N | 4.91 | 4.88 |

EXAMPLE 4: N-dodecylhexahydroazepine

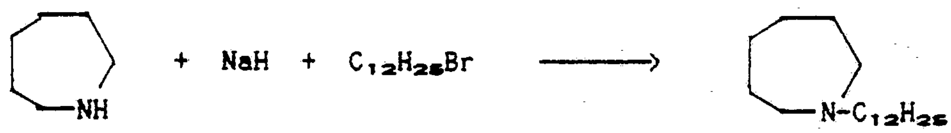


4 g (166.7 mmoles) of NaH (98%) are suspended in 350 ml of anhydrous toluene in a 1 litre flask fitted with a thermometer, reflux condenser and mechanical stirrer and kept under a nitrogen atmosphere. 15 g (151 mmoles) of azepine are then added in portions. The mixture is heated under reflux for about 1 hour. 37.6 g (151 mmoles) of n-dodecylbromide previously dissolved in 50 ml of toluene are then dripped in. After the addition the mixture is left boiling under reflux for 20 hours. The progress of the reaction is followed by TLC. On termination of the reaction the unreacted sodium hydride is filtered off and the toluene evaporated under reduced pressure. The residue is taken up in ethyl ether and washed several times with water. It is dried with $Na_2SO_4$ and the ethyl ether evaporated.

35 g (yield 86.6%) of product are obtained in the form of a straw yellow oil which can be further purified by distillation under reduced pressure or by chromatography on $SiO_2$.
I.R. (liquid film): $\nu_{C-H}$ 2920 $cm^{-1}$, 2850 $cm^{-1}$, 1465 $cm^{-1}$

| Elementary analysis: | CALCULATED | FOUND |
|---|---|---|
| C | 80.90 | 80.83 |
| H | 13.86 | 13.83 |
| N | 5.24 | 5.18 |

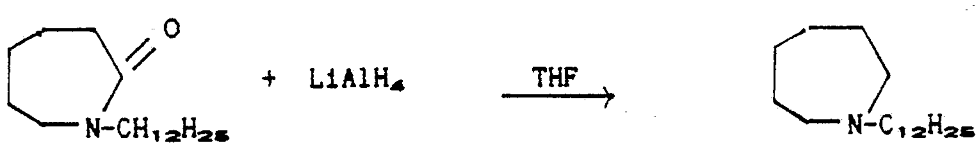


5 g (17.8 mmoles) of 1-dodecylazacycloheptan-2-one are diluted under a nitrogen atmosphere with 200 ml of anhydrous tetrahydrofuran in a 1 litre flask fitted with a thermometer and mechanical stirrer. 1.11 g (29.2 mmoles) of $LiAlH_4$ are added in portions to the resultant mixture.

The mixture is stirred for 2 hours at ambient temperature until the reaction is complete (TLC, eluent ethyl acetate, displayed by exposure to $I_2$ vapour).

On completion of the reaction the excess $LiAlH_4$ is destroyed by adding firstly ethyl acetate and then water dropwise.

The phases are separated, the organic phase is washed several times with water and dried with $Na_2SO_4$, after which the sulphate is filtered off and the solvent eliminated by distillation under reduced pressure.

The oil obtained is dissolved in ether and subjected to acid-base washing with $H_2SO_4$-NaOH.

Washing is repeated several times until a constant product is obtained under TLC.

2.8 g (yield 58.8%) of pure product are obtained in the form of straw yellow oil.

I.R. (liquid film): the disappearance of $\nu_{C=O}$ is verified.

EXAMPLE 5: Synthesis of 4-decanoylthiomorpholide

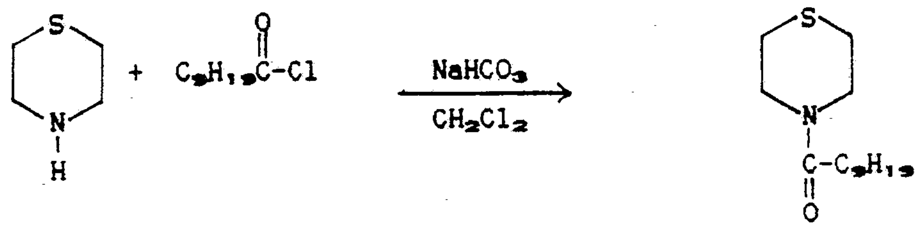


4.6 g (44.54 mmoles) of thiomorpholine are dissolved in 80 ml of methylene chloride in a water-bath cooled 250 ml flask fitted with a thermometer and magnetic stirrer, and 4.1 g (49 mmoles) of sodium bicarbonate are suspended in the resultant solution.

8.5 g (9.13 ml, 44.54 mmoles) of decanoyl chloride are dissolved separately in 20 ml of methylene chloride and this mixture is dripped slowly into the thiomorpholine solution over about 30 minutes.

After the addition is complete, the reaction mixture is left stirring at ambient temperature for about 1 hour, or until the disappearance of the flocky precipitate formed during the addition.

The progress of the reaction is followed by TLC ($SiO_2$; $CHCl_3$:$CH_3OH$ = 10:0.2), displaying the spots by exposure to $I_2$ vapour.

On completion of the reaction the crude product is washed several times with water, 6N HCl and again with water. The solvent is evaporated under reduced pressure to obtain 11 g (M.W. 257.46, yield 96%) of product in the form of colourless dense oil pure under TLC. B.P. 170 °C at 1 mmHg.

I.R. (liquid film): $\nu_{C=O}$ 1650 $cm^{-1}$

N.M.R. ($CDCl_3$, TMS): $\delta$ 0.95 (t, 3HJ. $CH_3$ alkyl);$\delta$1.4 (s, 14H, -$(CH_2)_7$-alkyl),$\delta$2.40 (t, 2H, $-\underset{\overset{\|}{O}}{C}-CH_2-$) ;$\delta$2.6-2.8 (m, 4H, thiomorpholine ring, H adjacent to S);$\delta$3.70-4.00 (m, 4H, thiomorpholine ring, H adjacent to N)

| Elementary analysis: | CALCULATED | FOUND |
|---|---|---|
| C | 65.25 | 65.13 |
| H | 10.49 | 10.44 |
| N | 5.44 | 5.37 |

EXAMPLE 6: Synthesis of 4-palmitoylthiomorpholide

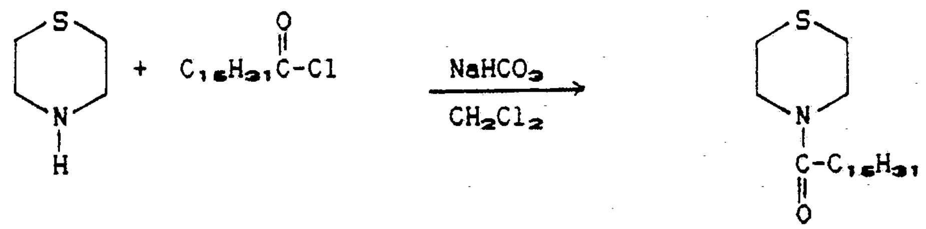


4.6 g (44.54 mmoles) of thiomorpholine are dissolved in 80 ml of methylene chloride in a water-bath cooled 250 ml flask fitted with a thermometer and magnetic stirrer, and 4.1 g (49 mmoles) of sodium bicarbonate are suspended in the resultant solution.

12.24 g (13.53 ml, 44.54 mmoles) of palmitoyl chloride are dissolved separately in 20 ml of methylene chloride and this mixture is dripped slowly into the thiomorpholine solution over about 30 minutes.

After the addition is complete, the reaction mixture is left stirring at ambient temperature for about 1 hour, or until the disappearance of the flocky precipitate formed during the addition.

The progress of the reaction is followed by TLC ($SiO_2$; $CHCl_3$:$CH_3OH$ = 10:0.2), displaying the spots by exposure to $I_2$ vapour.

On completion of the reaction the mixture is poured into a separator funnel where it is washed several times with water, 6N HCl and again with water. The organic solution is dried with $Na_2SO_4$ and the methylene chloride evaporated under reduced pressure.

14.36 g (M.W. 341.62, yield 94.3%) of microcrystalline white powder is obtained which is pure under TLC. M.P. 55-56.5 °C.

I.R. (nujol): $\nu_{C=O}$ 1640 $cm^{-1}$

N.M.R. ($CDCl_3$, TMS): δ 0.9 (t, 3HJ, $CH_3$alkyl);δ1.35 (s, 26H, -$(CH_2)_{13}$-alkyl),δ2.3 (t, 2H, - $\underset{\underset{O}{\|}}{C}$ -$CH_2$-) ;δ2.6-2.85 (m, 4H, thiomorpholine ring, H adjacent to S);δ3.7-4.05 (m, 4H, thiomorpholine ring, H adjacent to N)

| Elementary analysis: | CALCULATED | FOUND |
|---|---|---|
| C | 70.25 | 69.96 |
| H | 11.41 | 11.33 |
| N | 4.10 | 4.01 |

EXAMPLE 7: Synthesis of 4-lauroylmorpholide

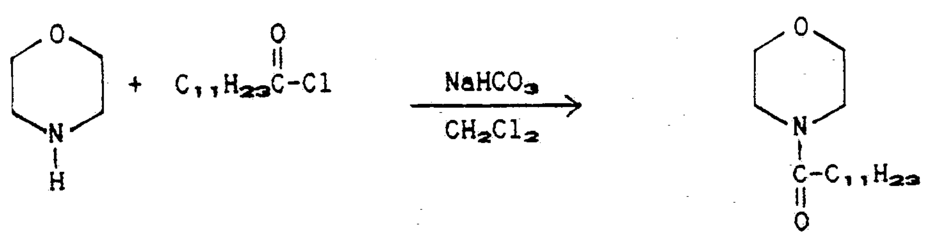


5 g (57.4 mmoles) of morpholine are dissolved in 100 ml of methylene chloride in a 250 ml flask fitted with a thermometer and magnetic stirrer, and 5.3 g (63.1 mmoles) of sodium bicarbonate are suspended in the resultant solution.

12.56 g (13.27 ml, 57.4 mmoles) of lauroyl chloride are dissolved separately in 20 ml of methylene chloride and this mixture is dripped slowly into the morpholine solution over about 30 minutes.

After the addition is complete, the reaction mixture is left stirring at ambient temperature until the disappearance of the precipitate which forms (about 1 hour).

The progress of the reaction is followed by TLC ($SiO_2$; $CH_2Cl_2$: $CH_3OH$ ratio 10 : 0.2), displaying the spots by exposure to $I_2$ vapour.

On completion of the reaction the mixture is poured into a separator funnel where it is washed several times with water, 6N HCl and again with water. The organic solution is dried with $Na_2SO_4$ and the methylene chloride evaporated under reduced pressure. 15.12 g (M.W. 269.44, yield 97.76%) of product are obtained in the form of an odourless, colourless dense liquid pure under TLC.

I.R. (liquid film): $\nu_{C=O}$ 1650 $cm^{-1}$

N.M.R. ($CDCl_3$, TMS): $\delta$ 0.90 (t, 3H, $CH_3$alkyl);$\delta$1.35 (s, 18H, -$(CH_2)_9$-alkyl),$\delta$2.38 (t, 2H, $-\underset{\underset{O}{\|}}{C}-CH_2-$) ;$\delta$3.35-3.75 (m, 8H, morpholine ring)

| Elementary analysis: | CALCULATED | FOUND |
|---|---|---|
| C | 71.26 | 70.99 |
| H | 11.51 | 11.37 |
| N | 5.19 | 5.08 |

Skin permeability tests using compounds of the present invention

This study was carried out by "in vitro" tests using a skin diffusion cell apparatus consisting of two glass segments separated by a layer of skin taken from a "nude" mouse, genetically without hair. Suspensions of the substance to be tested are deposited on the stratum corneum of the skin. The dermic layer dips into a buffer solution from which withdrawals are made at intervals to determine the quantity of substance which has passed through the epidermis.

The medicament for which passage through the skin is tested is naproxen-Na. 1-dodecylazacycloheptan-2-one (azone) is used as reference standard.

All the tested compounds are insoluble in aqueous solution.

Suspensions at the required concentrations (0.5 and 1% w/v) are then prepared by ultrasonic treatment three times for 30 seconds in an ice bath with a titanium-tipped ultrasonic generator. The pHs of the 1% suspensions in 0.1 M pH 7.0 phosphated buffer are as follows:

Table 1

| Compound | pH |
|---|---|
| Example 1 | 8.00* |
| Example 2 | 7.35* |
| Example 3 | 7.38* |
| Example 4 | 7.80** |
| azone | 7.80** |

* in the presence of 1% naproxen-Na
** in the presence of 5% naproxen-Na

The volume of examined suspension applied externally to the skin is 1 ml. The volume of buffer solution in the cell lower chamber is 10 ml. After each withdrawal of 1 ml of solution from the lower chamber, the volume was made up to 10 ml by adding buffer. This progressive dilution was taken into account in calculating the total nmoles of naproxen-Na which had passed.

The naproxen-Na in the buffer solution was determined by fluorimetric analysis.

Naproxen-Na when excited at 312 nm emits a fluorescence peak at 355.5 nm and the response varies linearly with concentration up to 200 nmoles/ml. Beyond this concentration the fluorescence is quenched, so that the samples have to be diluted before reading.

Two sets of experiments were carried out, the first relative to Tables 2, 3, 4 and 5 and the second relative to Tables 6, 7, 8 and 9.

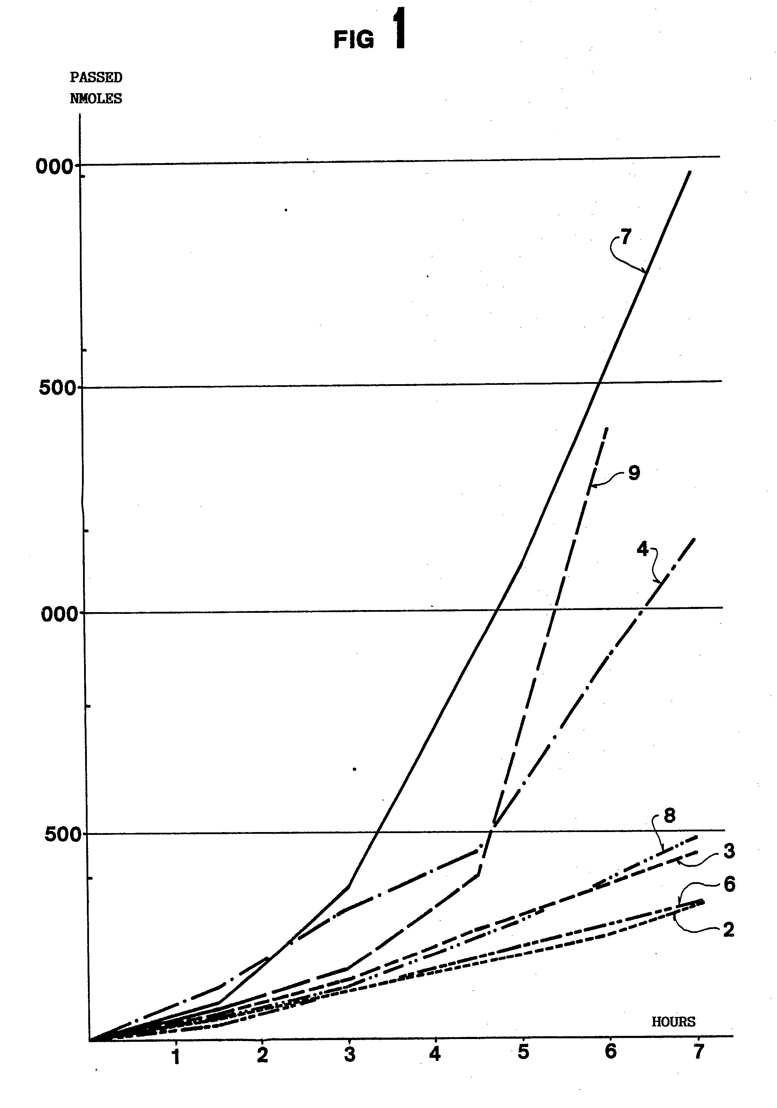

The test results are shown in the underlying tables and in the accompanying Figure 1.

Table 2

| NAPROXEN-Na (1%) | | |
|---|---|---|
| Time in hours | F.U.a. 355.5 nm | nmoles passed |
| 1.5 | 0.020 | 50 |
| 3.0 | 0.045 | 117 |
| 4.5 | 0.073 | 189 |
| 6.0 | 0.097 | 255 |
| 7.0 | 0.125 | 326 |

Table 3

| AZONE (1%) | | |
|---|---|---|
| Time in hours | F.U.a. 355.5 nm | nmoles passed |
| 1.5 | 0.021 | 52 |
| 3.0 | 0.055 | 143 |
| 4.5 | 0.107 | 276 |
| 6.0 | 0.144 | 379 |
| 7.0 | 0.174 | 454 |

Table 4

| COMPOUND EXAMPLE 3 (1%) | | |
|---|---|---|
| Time in hours | F.U.a. 355.5 nm | nmoles passed |
| 1.5 | 0.051 | 127 |
| 3.0 | 0.125 | 325 |
| 4.5 | 0.173 | 452 |
| 6.0 | 0.348 | 895 |
| 7.0 | 0.438 | 1159 |

Table 5

| COMPOUND EXAMPLE 3 (1%) | |
|---|---|
| Without naproxen - buffer alone | |
| Time in hours | F.U.a. 355.5 nm |
| 1.5 | 0.002 |
| 3.0 | 0.004 |
| 4.5 | 0.003 |
| 6.0 | 0.002 |
| 7.0 | 0.002 |

Table 6

| NAPROXEN-Na (1%) | | |
|---|---|---|
| Time in hours | F.U.a. 355.5 nm | nmoles passed |
| 1.5 | 0.015 | 37.5 |
| 3.0 | 0.044 | 114.0 |
| 5.0 | 0.092 | 238.0 |
| 7.0 | 0.128 | 336.0 |

Table 7

| COMPOUND EXAMPLE 2 (0.5%) | | |
|---|---|---|
| Time in hours | F.U.a. 355.5 nm | nmoles passed |
| 1.5 | 0.033 | 82.5 |
| 3.0 | 0.143 | 366.0 |
| 5.0 | 0.426 | 1093.0 |
| 7.0 | 0.755 | 1968.0 |

Table 8

| COMPOUND EXAMPLE 3 (0.5%) | | |
|---|---|---|
| Time in hours | F.U.a. 355.5 nm | nmoles passed |
| 1.5 | 0.013 | 32.5 |
| 3.0 | 0.051 | 131.0 |
| 5.0 | 0.114 | 295.0 |
| 7.0 | 0.187 | 487.0 |

Table 9

| COMPOUND EXAMPLE 4 (1%) | | |
|---|---|---|
| Time in hours | F.U.a. 355.5 nm | nmoles passed |
| 1.5 | | 75 |
| 3.0 | | 175 |
| 4.5 | | 400 |
| 6.0 | | 1410 |

The compound of Example 1, when tested under the same conditions as the compound of Example 3 at a concentration of 1%, gives practically equal results.

Figure 1 shows the results of tables 2, 3, 4, 6, 7, 8 and 9 in graphical form. In this figure the individual curves are indicated by the number of the corresponding table.

From the tables and the respective curves it can be seen that:

- when used alone without any facilitating substance, naproxen-Na has very poor passage power (Tables 2 and 6);
- when using azone as facilitating substance a modest increase in passage power is obtained (Table 3);
- when using compounds of the present invention as facilitating substances at the same concentration as azone, the increase in passage power is very high (Tables 4. 7 and 9).

Table 5 shows that the fluorescence values represent a true measure of the amount of naproxen-Na which has passed. In this respect, from this table it can be seen that the compounds of the invention, when applied alone to the skin, do not contribute even minimally to fluorescence.

Anti-inflammatory and antiallergic activity tests on the compounds of the present invention

"In vitro" determinations of antiserotonin, antihistaminic and $LTD_4$ antagonist activity were made on the compound of Example 5. The tests were carried out by the following methods:

Test 37 - $LTD_4$ antagonist (in vitro):

Test substance (mcg/ml) inhibition of submaximal contractile responses of isolated guinea pig ileal segments, bathed in physiological salt solution at 37° C, to $LTD_4$ (10 ng/ml) by more than 50 percent (>50) indicates significant activity.

Test 73 - Antihistaminic (in vitro):

Inhibition of submaximal contractile responses of isolated guinea pig ileal segments, bathed in physiological salt solution, to histamine (0.5 mcg/ml) by more than 80 percent (>80) indicates activity.

Test 74 - Antiserotonin (in vitro):

Test substance (mcg/ml) inhibition of submaximal contractile response of isolated guinea pig ileal segments, bathed in physiological salt solution, to serotonin (1 mcg/ml) by more than 80 percent (>80) indicates activity. The results of these tests are shown in the Table 10.

TABLE 10

| No. | Test | Reference compound | Route | Dose γ/ml | Response % inhibition |
|---|---|---|---|---|---|
| 37 | $LTD_4$ antagonist | | in vit | 8 | 75 |
| | " | | " | 4 | 28 |
| | " | FLP-55712 | " | 0.05 | 60 |
| 73 | Antihistaminic | | in vit | 10 | 100 |
| | " | | " | 2.5 | 82 |
| | " | | " | 1 | 18 |
| | " | Terfenadine | " | 25 | 83 |
| 74 | Antiserotonin | | in vit | 10 | 100 |
| | " | | " | 2.5 | 100 |
| | " | | " | 1 | 100 |
| | " | | " | 0.5 | 68 |
| | " | Cyproheptadine | " | 1 | 85 |

Toxicity of the compounds of the present invention

A further interesting characteristic of the compounds of the present invention is their low toxicity. The $LD_{50}$ values in the mouse in fact exceed 6000 mg/kg by oral administration.

By virtue of the illustrated characteristics, the compounds of the present invention find useful application as active principles in pharmaceutical compositions able to facilitate passage of active substances through physiological barriers.

They also find application in pharmaceutical compositions for external topical use for anti-inflammatory and antiallergic purposes, possibly mixed with other active or inactive substances.

**Claims**

1. N-substituted aliphatic heterocyclic compounds having the following general formula:

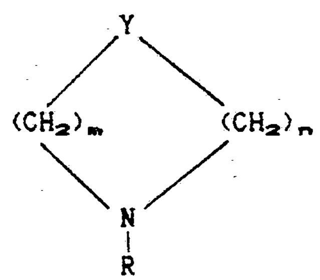

(I)

where Y is $CH_2$, S; S->0 or 0;
m and n are whole numbers between 0 and 10 and such that their sum is a whole number between 3 and 10; and
R is a $C_6$-$C_{20}$ linear or branched alkyl or an acyl of general formula
$-\overset{O}{\overset{\|}{C}}-R_1$, in which $R_1$ is a $C_5$-$C_{19}$ linear or branched alkyl.

2. A process for preparing N-substituted aliphatic heterocyclic compounds having the following general formula:

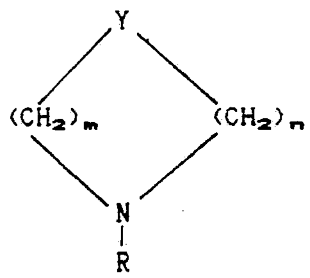

(I)

where Y is $CH_2$, S, S->0 or 0;
m and n are whole numbers between 0 and 10 and such that their sum is a whole number between 3 and 10; and
R is a $C_6$-$C_{20}$ linear or branched alkyl or an acyl of general formula
$-\overset{O}{\overset{\|}{C}}-R_1$, in which $R_1$ is a $C_5$-$C_{19}$ linear or branched alkyl, characterised by treating the compound

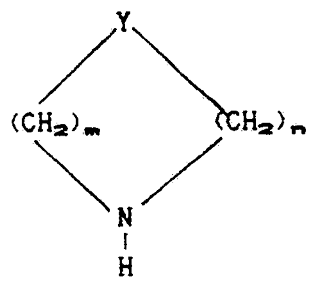

(II)

in an organic solvent means with NaH and then with an RX alkyl halide, or treating (II) with an $R_1-\overset{O}{\overset{\|}{C}}-X$ acyl halide in the presence of an alkaline substance, where R and $R_1$ have the aforesaid meanings and X is a halogen.

3. A process as claimed in claim 2, characterised in that said treatment with NaH is conducted in toluene while boiling under reflux.

4. A process as claimed in claim 2, characterised in that said treatment with the RX alkyl halide is conducted in toluene while boiling under reflux.

5. A process as claimed in claim 2, characterised in that said RX alkyl halide is an alkyl bromide.

6. A process as claimed in claim 2, characterised in that said treatment with the $R_1-\overset{O}{\overset{\|}{C}}-X$ acyl halide is conducted in benzene or in methylene chloride at ambient temperature.

7. A process as claimed in claim 2, characterised in that said alkaline substance is sodium bicarbonate.

8. A process as claimed in claim 2, characterised in that said acyl halide is an acyl chloride.

9. Pharmaceutical compositions containing at least one compound of general formula (I) for the purpose of facilitating passage of active substances through physiological barriers.

10. Pharmaceutical compositions containing at least one compound of general formula (I) for external topical use for anti-inflammatory and antiallergic purposes, possibly mixed with other active or inactive substances.

Claims for the following Contracting States: GR, ES

1. A process for preparing N-substituted aliphatic heterocyclic compounds having the following general formula:

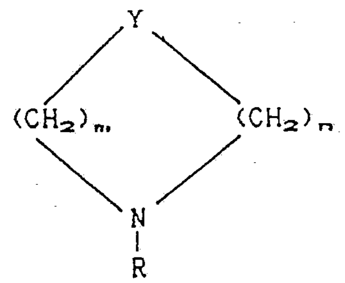

(I)

where Y is $CH_2$, S, S->0 or 0;
m and n are whole numbers between 0 and 10 and such that their sum is a whole number between 3 and 10; and
R is a $C_6$-$C_{20}$ linear or branched alkyl or an acyl of general formula $-\overset{O}{\overset{\|}{C}}-R_1$, in which $R_1$ is a $C_5$-$C_{19}$ linear or branched alkyl, characterised by treating the compound

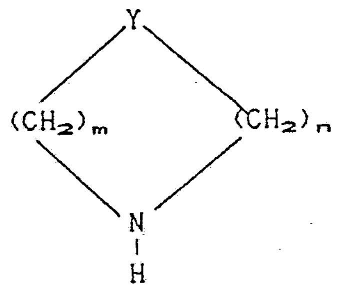

(II)

in an organic solvent means with NaH and then with an RX alkyl halide, or treating (II) with an $R_1-\overset{O}{\overset{\|}{C}}-X$ acyl halide in the presence of an alkaline substance, where R and $R_1$ have the aforesaid meanings and X is a halogen.

2. A process as claimed in claim 1, characterised in that said treatment with NaH is conducted in toluene while boiling under reflux.

3. A process as claimed in claim 1, characterised in that said treatment with the RX alkyl halide is conducted in toluene while boiling under reflux.

4. A process as claimed in claim 1, characterised in that said RX alkyl halide is an alkyl bromide.

5. A process as claimed in claim 1, characterised in that said treatment with the $R_1-\overset{O}{\overset{\|}{C}}-X$ acyl halide is conducted in benzene or in methylene chloride at ambient temperature.

6. A process as claimed in claim 1, characterised in that said alkaline substance is sodium bicarbonate.

7. A process as claimed in claim 1, characterised in that said acyl halide is an acyl chloride.

FIG 1